# EUROPEAN PATENT SPECIFICATION

(11) **EP 1 970 012 B1**
(45) Date of publication and mention of the grant of the patent: **02.11.2011**
(21) Application number: 08102428.3
(22) Date of filing: 10.03.2008
(51) Int. Cl.: A61B 17/02, A61B 17/34

(54) **Trocar for thoracic surgery**
Trokar für die Thoraxchirurgie
Trocart pour chirurgie thoracique

(30) Priority: 14.03.2007 IT FI20070060
(43) Date of publication of application: 17.09.2008
(73) Proprietor: AB Medica S.p.A., 20020 Lainate (MI) (IT)
(72) Inventor: Ferrari, Danilo, 52100, Arezzo (IT)
(74) Representative: Bardini, Marco Luigi

(56) References cited:
- WO-A-2006/117819
- GB-A- 330 629
- US-A1- 2005 165 281
- US-A1- 2006 052 672
- US-A1- 2007 010 716

## Description

The present invention generally relates to the field of the instrumentation and devices for endoscopic surgery, and more precisely it relates to a trocar especially designed for thoracic surgery.

As is known, thoracic surgery has been increasingly developing in the direction of much reduced invasiveness, in particular as regards cardiac interventions. This trend is made possible by an endoscopic surgery technique which developed mainly for abdominal cavity operations, the so-called laparoscopic surgery. This technique provides for the use of devices, called "trocars", that generate and maintain access paths for the various surgical instruments to the body cavity where the operation takes place. The trocars are in practice formed by a cannula having side abutment means for engaging against the walls of the body opening made by the surgeon to access the internal cavity.

The trocars used for laparoscopic surgery are different from the trocars used in thoracic surgery. In the first case, in fact, it is normal practice to blow gas into the abdominal cavity, so to dilate and stretch out its tissues in order to facilitate internal vision. For this reason, the trocars for laparoscopic surgery are equipped both with valves that prevent the gas from escaping therefrom, and with anchoring means for securing the trocar to the side walls of the opening in which the trocars are inserted and opposing the expulsion thrust caused by the presence of gas in the cavity.

In thoracic surgery there is no need to insert gas inside the cavity to be operated (or at least there is no need to keep such cavity pressurised), and moreover the overall tissue thickness to be crossed in order to reach the cavity is lower than the tissue thickness to be crossed when the abdominal cavity has to be reached. For this reason, the trocars for thoracic surgery are structurally much simpler and compact with respect to the trocars for laparoscopic surgery.

In order to minimise, as much as possible, the invasiveness of the incision in which the trocar is inserted, the trocars are made of small dimensions and are subsequently enlarged (by virtue of the tissue elasticity) by means of a retractor.

The surgeon is thus obliged to carry out the trocar insertion in different steps, a first step in which a retractor is inserted in the cavity access incision, a second step in which the incision is enlarged due to the action of the retractor and a third step in which the trocar is inserted while the retractor is extracted.

WO2006/11781 discloses a trocar for laparoscopic surgery, comprising a guide duct for surgical instruments adapted to be inserted in a body opening of access to the abdominal cavity. The duct is formed by a plurality of sectors arranged around its axis and articulated to a support frame, sector expansion means being associated with the duct for mutually displacing the sectors from an initial insertion position in the body opening to a final, maximum expansion position in which they are moved away from the axis to a greater extent than in the configuration assumed in the initial position. The expansion means comprise a control ring nut movably connected with the sectors and the support frame, such that a rotation of the ring nut in one direction, called the expansion direction, results in a movement of the sectors away of said axis.

The sectors are integral to respective arms hinged to the support frame and the connection between the ring nut and the arm is configured to cause the arms to rotate around their hinge axis when the ring nut is rotated around its own axis.

US 2005/165281, US 2007/010716 and US 2006/052672 all disclose surgical retractors in which the guide duct is formed by a plurality of sector. In these devices the sectors are integral to a slide engaged with radial racks or threaded arms. Therefore, the movement of the sectors away from the duct axis is the result of a linear sliding.

GB 330629 discloses an expansible speculum comprising a number of blades extending axially from a housing containing a mechanism adapted for simultaneously operating the blades to spread them in several directions from the axis, so that a passage, into which the blades are inserted, is uniformly expanded.

The mechanism for spreading the blades is of the type in which the operation of a screw or knob in a circular direction effects an equal and parallel outward movement of all the blades.

The object of the present invention is to provide a trocar for thoracic surgery that permits simplifying the step of its insertion into the thoracic cavity access incision.

A particularly important object of the invention is to provide a trocar for thoracic surgery of the above mentioned type that permits the incision enlargement step and the trocar insertion step to be unified.

Another important object of the invention is to provide a' trocar for thoracic surgery of the above mentioned type that is structurally simple and easy to use.

These and other objects, which will be clearer below, are attained by a trocar for thoracic surgery comprising a guide duct for surgical instruments, the duct being adapted to be inserted in a body opening for access to the thoracic cavity, and formed by a plurality of sectors arranged around its axis and articulated to a support frame, sector expansion means being associated with the duct for mutually displacing the sectors from an initial insertion position in the body opening to a final maximum expansion position in which they are moved away from said axis to a greater extent than in the configuration assumed in the initial position, said expansion means comprising a control ring nut movably connected to the sectors and to the support frame, such that a rotation of the ring nut in one direction, called the expansion direction, results in a movement of the sectors away of the axis, means for locking the rotation in the opposite direction with respect to the expansion direction being associated with the ring nut, the rotation locking means comprising a temporary removal device of the rotation hindrance of the ring nut in the direction opposite the expansion direction, whereby the sectors can move backward to the initial position.

Other characteristics and advantages of the trocar according to the present invention will be apparent from the following description of an embodiment thereof, made as a non-limiting example with reference to the attached drawings, wherein:

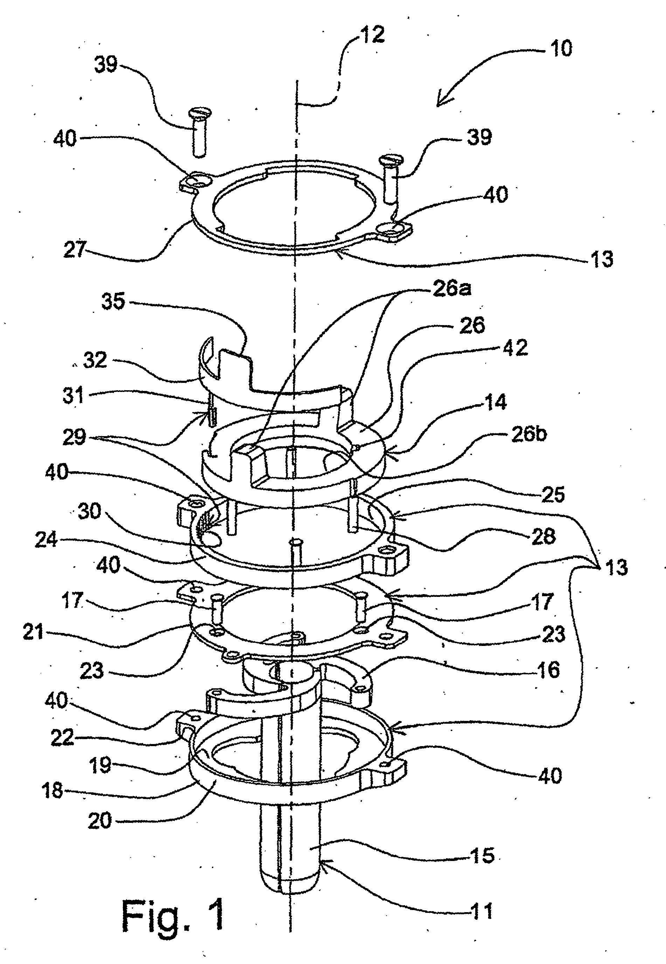

- Figure 1 shows an exploded view of the trocar according to the invention;
- Figure 2 shows an axonometric view of the trocar of figure 1 with expansion sectors in the initial insertion position in the body opening for accessing the thoracic cavity;
- Figure 3 shows a cross sectional view of the previous trocar shown in the figures, with the expansion sectors in its initial position;
- Figure 4 shows an axonometric view of the trocar shown in the previous figures, with the expansion sectors in its final, maximum expansion position;
- Figure 5 shows a cross sectional view of the trocar in the condition shown in figure 4;
- Figure 6 shows a further cross sectional view of the trocar shown in the previous figures, with the expansion sectors in its initial position and with a ratchet mechanism for keeping the sectors in stable intermediate expansion positions between their initial and final positions;
- Figure 7 shows an enlarged view of a portion of the cross section of the trocar of figure 6 relevant to the ratchet mechanism;
- Figure 8 shows a portion of the trocar along a cross section analogous to that of figure 7 showing the ratchet mechanism when the sectors are in their final, maximum expansion position;
- Figure 9 shows a longitudinal sectional view of the trocar of the previous figures, with the expansion sectors in the initial position and with an accessory element for locking the expansion sectors during the insertion step in the opening for accessing the body cavity;
- Figure 10 shows a cross section of the trocar with the expansion sector locking element as in figure 9;
- Figure 11 shows an axonometric view of the trocar of the preceding figures, with the expansion sectors in the initial position and with a key for facilitating the movement of the control ring nut when positioning the expansion sectors.

With reference to the aforesaid figures, a trocar for thoracic surgery according to the invention is generally indicated with 10 and comprises a guide duct T for surgical instruments adapted to be inserted in a body opening for accessing to the thoracic cavity. Duct T comprises a plurality of sectors 11 arranged around the axis 12 of the duct T and articulated to a support frame 13.

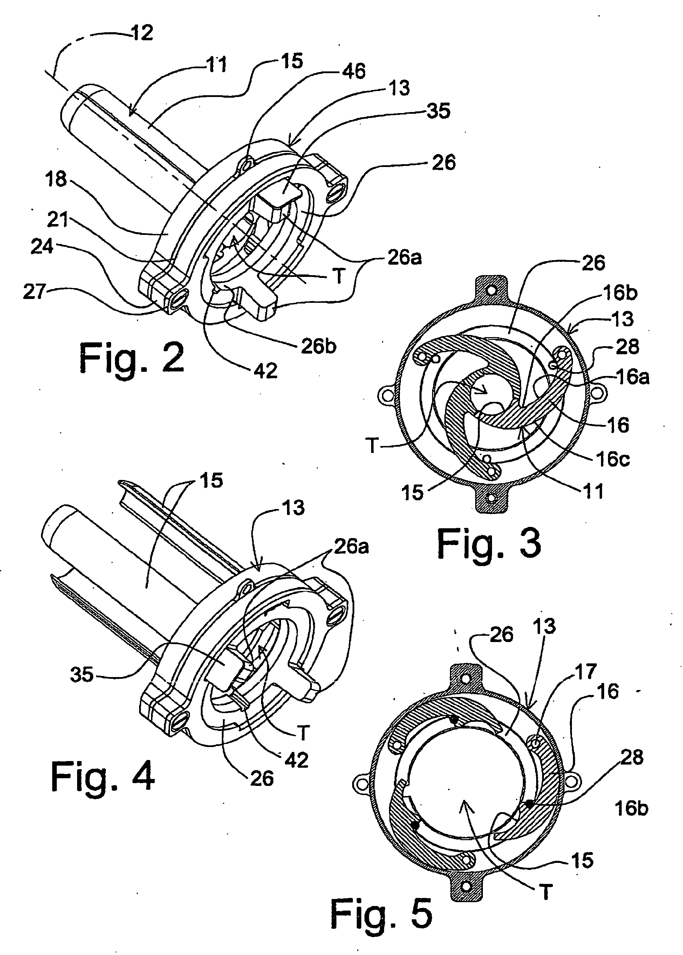

Associated with such duct T are expansion means 14 (described below) from an initial insertion position in the body opening (see figures 2 and 3) to a final, maximum expansion position (see figures 4 and 5) in which the sectors are moved away from the axis 12 to a greater extent than in the configuration assumed when they are in the initial position.

Each sector 11 comprises a longitudinal body 15 extending parallel to the axis 12. A preferably curved arm 16, connected at a free end to the frame 13 by means of a hinge pin 17, extends from one end of the longitudinal body 15 and on a plane orthogonal to the axis 12. It has to be noted that, when the sectors 11 are in their initial position, the longitudinal bodies 15 form a cylindrical duct closed along its own side surface.

In the present embodiment, the frame 13 comprises a first annular element 18 having an internal base 19, on which the arms 16 of the sectors 11 are slidingly housed, and a lateral containment wall 20 for the curved arms 16.

The frame 13 also comprises a second annular element 21, fixed to the upper edge 22 of the wall 20 of the first annular element 18. The hinge pins 17 for the arms 16 are connected to the second annular element 21 and, in particular, pins 17 are partially inserted in through holes 23 formed thereon. A third annular element 24 of the frame 13 is fixed to the second annular element 21 on the opposite side relative to the first annular element 18 and has an internal cylindrical side surface 25 that defines a rotational housing space for a control ring nut 26, for controlling the movement of the sectors 11. The control ring nut 26 is locked in the movement along the axis 12 between the second annular element 21 and a fourth annular element 27 fixed to the edge of the third annular element 24 on the opposite side with respect to the second annular element 21.

The expansion means 14, which provide spreading out or opening out of the sectors 11 comprise the control ring nut 26 and on the surface of the nut 26 facing towards the second annular element 21 pins 28 are provided that extend until they come into contact with the inner sides 16a, i.e. the concave sides facing towards the axis 12, of respective arms 16. Following a rotation of the control ring nut 26 in one direction, from hereafter referred to as expansion direction, the pins 28 are adapted to slide along the inner sides 16a in order to permit the opening movement of the sectors 11 from the initial insertion position (figure 2) in the body opening for accessing to the thoracic cavity, to the final, maximum expansion position (figure 4). On inner sides 16a, at the attachment end zone of the arms 16 to the longitudinal bodies 15, end stop abutments 16b are formed for the pins 28 when the sectors 11 are in the maximum expansion position.

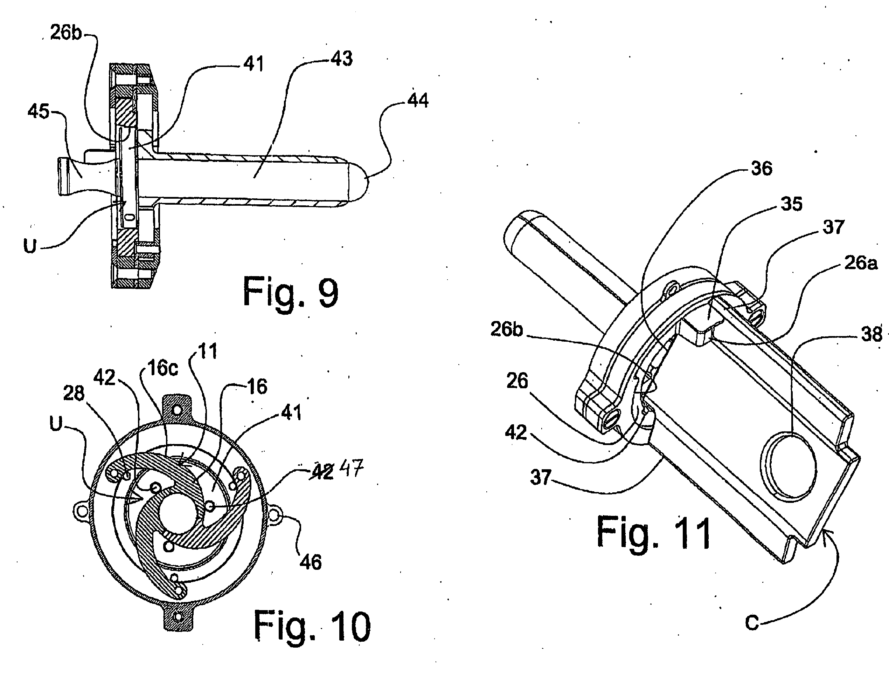

The ring nut 26 has, on the surface opposite that where the pins 28 are formed, two diametrically opposed reliefs 26a that constitute grip elements for the surgeon's hand or abutment elements for an accessory element, such as a key C that allows the movement of the control ring nut, as shown in figure 11 and as will be better explained below.

Associated with the control ring nut 26 are means 29 for locking the rotation of the same ring nut in the direction opposite the expansion direction. In this embodiment, the means 29 for locking the rotation of the ring nut 26 are, for example, a ratchet mechanism interposed between the support frame 13 and the ring nut 26. In particular, the ratchet mechanism comprises a toothed portion 30 (with saw tooth shape), defined on the inner side surface 25 of the third annular element 24, and a pawl 31 elastically engageable with the toothed portion 30. The pawl 31 is for example formed by an elastic plate fixed at one end to the cylindrical side of the ring nut 26, the other end being free to slide on the toothed portion 30, in the direction permitted by the slope of the teeth.

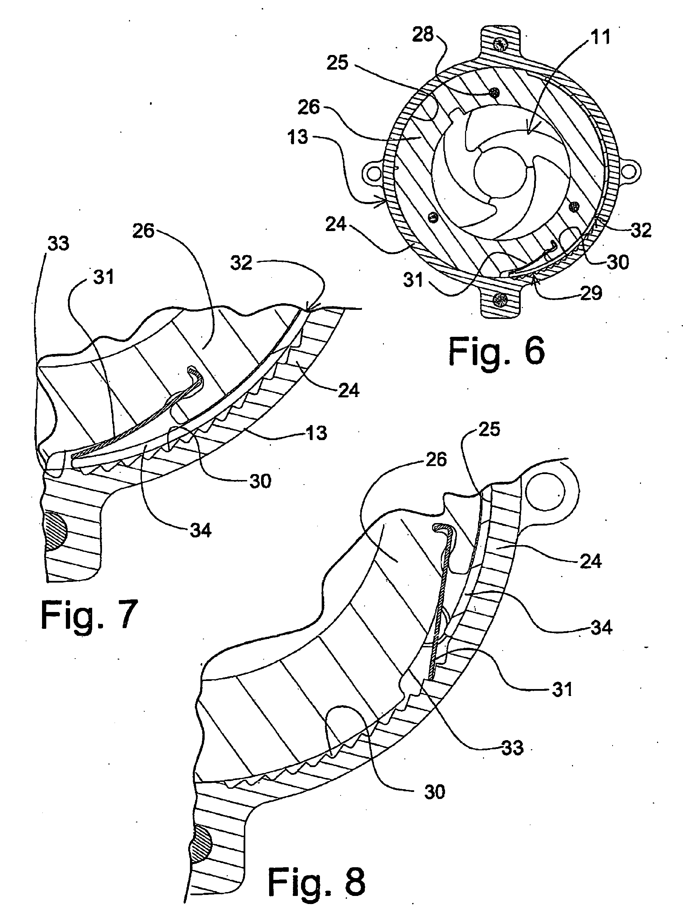

The rotation locking means 29 moreover comprise a device 32 for temporarily removing the ring nut rotation hindrance that, with reference to the ratchet mechanism just described, is formed by a disengagement device of the pawl 31 from the toothed portion 30. As is clearly visible in figures 7 and 8, the pawl 31 is arranged in a recess 33 on the cylindrical flank of the ring nut 26. The device 32 consists of a tongue 34, slidably arranged between the cylindrical flank of the ring nut 26 and the inner side surface 25 of the third annular element 24. Depending on the angular position taken along the cylindrical flank of the ring nut 26, the tongue 34 can be interposed between the free end of the elastic plate forming the pawl 31 and the toothed portion 30, thus permitting the disengagement of the pawl. The tongue 34 has a control appendage 35 that projects in the direction opposite the extension of the longitudinal bodies 15. The control appendage 35 is provided at one of the two reliefs 26a, and faces thereto.

It is noted how the width of the control appendage is greater than the width of the corresponding relief 26a.

In the maximum opening position of the trocar, or the final, maximum expansion position of the sectors 11, the trocar is in stable equilibrium and cannot be spontaneously closed due to the radial pressure exerted by the expanded tissues, while in the partial opening configurations, the ratchet mechanism prevents the spontaneous closure due to said pressure.

In order to close the trocar and bring the sectors 11 back into the initial insertion position in the opening made in the tissues to access the thoracic cavity, it is necessary to move the tongue 34. The tongue 34 can carry out a short rotation relative to the ring nut 13 by passing from the locking position (figure 8) to the release position (figures 6 and 7). In the locking position, the elastic plate forming the pawl 31 is free to engage on the toothed portion 32 of the third annular element 24, while in the release position the tongue 34 causes the elastic plate to be bent, forcing it to abandon the grip on the toothed portion 31 and rearranging it inside the recess 33.

In order to expand the trocar, it is necessary to rotate the ring nut 26 in anticlockwise direction by operating on reliefs 26a by hand or with the suitable key C of figure 11. Since the control appendage 35 of the tongue 34 has a width greater than that of the corresponding relief 26a to which it faces, the right side of the control appendage 35 is first aligned with the right side of the relief 26a of the ring nut 26, bringing the tongue 34 from a release position, in which it is interposed between the pawl 31 and the toothed portion 30, to the locking position, in which the pawl 31 is free to engage on the saw teeth. A further rotation of the key (or hand) causes the pins 28 of the ring nut 26 to push the arms 16 towards the outside, with consequent moving away of the sectors 11 from the axis 12. The sectors will always remain in expanded state thanks to the ratchet mechanism, when in operation.

The key C has an end portion 36 adapted for being inserted in the hole 26b of the ring nut 26 and two side abutment portions 37 that cooperate with the control appendage 35 and the reliefs 26a. Moreover the key C has a hole 38 through which the surgeon distinguishes by the touch the grip side for the opening of the trocar from the grip side for the closing.

The annular elements forming the frame 13 are connected by threaded connections passing through the annular elements, such as for example a pair of opposing screws 39 passing through corresponding eyelets 40 formed peripherally on the annular elements 18, 21, 24 and 27.

The expansion of the sectors 11 is in practice monodirectional, as the three longitudinal bodies 15 are free to be moved away from the axis 12 but not to return back, by virtue of the ratchet mechanism. This is not a problem when the trocar is inserted in the patient's thorax, as sector expansion is opposed by the radial forces exerted by the elastic resistance of the patient tissues, but could constitute a drawback on the trocar insertion step, as a possible abutment contact could generate radial thrusts causing the sectors to expand before the insertion. Therefore, in order to avoid such risk, a further accessory element is provided, like an inserter tool U for preventing the expansion of the sectors 11 from the initial insertion position. This tool is usually associated with the trocar on the insertion step in the body opening and is subsequently removed before starting the expansion step.

In particular, in this embodiment, such inserter tool U, visible in figures 9 and 10, has a plate 41 with a shape complementary to the hole of the ring nut 26 and adapted to be inserted therein. Pins 47 are provided on the side of the plate 41 facing towards the sectors 11, each pin abutting against the external flank 16c of a respective arm 16, opposite the internal flank 16a on which the pins 28 of the ring nut 26 slide. The inserter tool U is secured to the ring nut 26 by means of a locking and positioning element (not shown in the drawings) projecting from the side of the plate 41 and adapted to be coupled with a groove 42 formed on the inner side surface of the ring nut 26. Advantageously, a rod-like portion 43 extends from the plate 41, in such a way to occupy the space defined between the longitudinal bodies 15 forming the sectors 11. The rod-like portion 43 projects from the duct T formed by sector 11 with one sphere-shaped end 44 that makes the insertion of the trocar in the body opening easier. On the opposite side, the plate 41 has a grip handle 45.

In order to prevent the trocar from slipping outside the opening made in the patient's thorax during the operation, the surgeon can secure the trocar to the thorax by means of fixing means, such as for example small rings 46 formed along the outside of the frame 13 and through which the surgeon can pass suture points for anchoring them to the patient's skin. Alternatively, the longitudinal bodies 15 forming the expansion sectors can be made with external surface with increased friction, by means of saw tooth corrugations or threads (elements not shown in the drawings for the sake of simplicity).

It is evident how the trocar described here is easily insertable in the body opening of access to the thoracic cavity, as during the insertion, the trocar sectors defining the access duct to the thoracic cavity have a very limited axial size. When the trocar is inserted in the thorax, the sectors can radially expand by enlarging the thoracic cavity access opening in order to permit the easy insertion of the surgical instruments.

In practice, a trocar and a retractor have been joined in a single instrument. Thus the number of tools to be used is reduced and the trocar insertion process simplified, entirely to the advantage of the surgeon who does not need to use additional instruments while the preliminary operating steps of the operation are reduced.

The trocar according to the invention can be subject to numerous modifications and variants, all being within the scope of the invention; moreover, all details can be replaced by other technically equivalent elements, without departing from the scope of the invention.

In practice, the materials used (provided that they are compatible with the specific use) as well as the size can be of any type according to technical requirements and the state of the art.

Where any of the characteristics and techniques mentioned in any claim are followed by a reference number, these have been included as an example with the sole object of increasing the clarity of the claims, and consequently they have no limiting effect on the interpretation of each element identified thereby.

## Claims

1. Trocar for thoracic surgery, comprising a guide duct (T) for surgical instruments adapted to be inserted in a body opening of access to the thoracic cavity, said duct (T) being formed by a plurality of sectors (11) arranged around its axis (12) and articulated to a support frame (13), sector expansion means (14) being associated with said duct (T) for mutually displacing said sectors (11) from an initial insertion position in the body opening to a final, maximum expansion position in which they are moved away from said axis (12) to a greater extent than in the configuration assumed in said initial position, said expansion means (14) comprising a control ring nut (26) movably connected with said sectors (11) and said support frame (13), such that a rotation of said ring nut (26) in one direction, called the expansion direction, results in a movement of the sectors (11) away of said axis (12), **characterized in that** means for locking the rotation (29) of said ring nut (26) in the direction opposite that of said expansion direction are associated with said ring nut (26), said rotation locking means (29) comprising a device (32) for temporarily removing the rotation hindrance of the ring nut (26) in the direction opposite the expansion direction, whereby the sectors (11) can move backwards to said initial position.

2. Trocar according to claim 1, **characterised in that** the rotation locking means (29) of said control ring nut (26) in the expansion direction comprise a ratchet mechanism interposed between said frame (13) and said control ring nut (26).

3. Trocar according to claim 2, **characterised in that** said ratchet mechanism comprises a toothed portion (30) with saw tooth profile formed on said frame, a pawl (31) elastically associated with said control ring nut (26), one end of the pawl (31) being free to slide on said toothed portion (30) in the direction permitted by the slope of the teeth, said device (32) for temporarily removing the rotation hindrance of the ring nut (26) in the direction opposite the expansion direction comprising a disengagement device of said pawl (31) from said toothed portion (30).

4. Trocar according to claim 3, **characterised in that** said frame (13) infernally defines a rotational housing space for said control ring nut (26), said toothed portion (30) with saw tooth profile being formed on the inner side surface (25) of said housing space for said control ring nut (26), said pawl (31) being formed by an elastic plate with one end fixed to the cylindrical flank of said control ring nut (26) and with the other end free to slide on said toothed portion (30) in the direction permitted by the slope of the teeth, said device (32) for disengaging the pawl (31) from the toothed portion (30) comprising a tongue (34) slidably arranged between the cylindrical flank of the control ring nut (26) and said inner side surface (25) of the frame (13), said tongue (34) being adapted to be interposed between the free end of the elastic plate forming the pawl (31) and said toothed portion (30) according to the angular position along the cylindrical flank of the control ring nut (26), thus permitting the disengagement of the pawl (31).

5. Trocar according to claim 4, **characterised in that** said control ring nut (26) has at least one relief (26a) constituting either a grip element for the surgeon's hand or abutment element for a driving key (C) of the ring nut (26), said at least one relief (26a) extending in the direction opposite the extension direction of said sectors (11), said disengagement tongue (34) of the pawl (31) of the ratchet mechanism having a control appendage (35) provided at said at least one relief (26a) and facing the latter (26a), the width of said control appendage (35) being greater than the width of the corresponding said at least one relief (26a).

6. Trocar according to any one of the preceding claims, **characterised in that** each of said sectors (11) comprises a longitudinal body (15) that is extended in the same direction of said axis (12), an arm (16) articulated at one free end to said frame (13) by means of a hinge pin (17) extending from one end of said longitudinal body (15), on a plane substantially orthogonal to said axis (12).

7. Trocar according to claim 6, **characterised in that**; when said sectors (11) are in the initial insertion position, said longitudinal bodies (15) form a cylindrical duct that is substantially closed along its own side surface.

8. Trocar according to claim 6 or 7, **characterised in that** each arm (16) is curved with concavity turned towards said axis (12).

9. Trocar according to any one of the claims 6 to 8, **characterised in that** said frame (13) is annular and surrounds said sectors (11), said frame (13) comprising
- a first annular element (18) having an inner base (19) on which the arms (16) of said sectors (11) slidably abut, and a lateral containment side (20) for said arms (16),
- a second annular element (21), fixed on the upper edge (22) of said side (20) of said first annular element (18), to which (21) hinge pins (17) for the arms (16) are bonded,
- a third annular element (24) fixed on said second annular element (21) on the part opposite the first annular element (18), said third annular element (24) having a cylindrical inner side surface (25) that defines a rotational housing space for said control ring nut (26),
- a fourth annular element (27) fixed on the edge of the third annular element (24) on the side opposite the second annular element (21), said control ring nut (16) being locked in movement along the axis (12) between the second annular element (21) and the fourth annular element (27).

10. Trocar according to claim 9, **characterised in that** the joining of said first, second, third and fourth annular element (18, 21, 24, 27) forming the frame (13) is achieved by two opposing screws (39) passing through corresponding eyelets (40) made peripherally on the same annular elements (18, 21, 24, 27).

11. Trocar according to any one of the claims 4 and 9, **characterised in that** said toothed portion (30) of the ratchet mechanism is formed on the inner side surface (25) of said third annular element (24) of the frame (13).

12. Trocar according to any one of the claims 6 to 10, **characterised in that** said expansion means (14) comprise pins (28) that are extended from the surface of said control ring nut (26) up to contact with the inner flanks (16a) of said arms (16), said pins (28) being slidable on said flanks (16a) to allow the opening movement of said sectors (11) from the initial insertion position in the body opening for accessing to the thoracic cavity to the final maximum expansion position.

13. Trocar according to claim 12, **characterised in that** on said inner flanks (16a) of said arms (16), at the attachment end zone of the arms (16) to the longitudinal bodies (15) forming said sectors (11), end stop abutments (16b) are formed for said pins (28) when the sectors (11) are in the maximum expansion position.

14. Trocar according to any one of the preceding claims, **characterised in that** it comprises fixing means to the patient's thorax.

15. Trocar according to claim 14, **characterised in that** said fixing means comprise small rings (46) made along the outside of said frame (13) adapted to permit the passage of suture points for anchoring to the patient's skin.

16. Trocar according to one or more of the preceding claims, **characterised in that** it comprises an accessory element constituted by an inserter tool (U) adapted to lock the expansion of said sectors (11) in the initial insertion position, said inserter tool. (U) being associable with the trocar body (10) only in the insertion step in the body opening and being subsequently removed before beginning the expansion step.

17. Trocar according to claim 16, **characterised in that** said inserted tool (U) has a plate (41) countershaped to the hole (26b) of said control ring nut (26) and fit for being inserted therein (26b), said plate (41) having, on the face turned towards said sectors (11), pins (47) adapted to abut against the external flanks (16c), opposite the internal flanks (16a) on which the pins (28) of said control ring nut (26) slide, of respective arms (16), said inserter tool (U) being locked on said control ring nut (26) by means of a locking and positioning element projecting from the side of said plate (41) and adapted to be coupled with a corresponding groove (42) made on the inner side surface of said control ring nut (26).

18. Trocar according to claim 17, **characterised in that**, a rod-like portion (43) extends from said plate (41) in the space defined between the longitudinal bodies (15) forming said sectors (11), said rod-like portion projecting from the duct formed by these (11) with a shaped end (44) to make easier the insertion of the trocar in the body opening.

19. Trocar, according to any one of the preceding claims, **characterised in that** it comprises an accessory element formed by a key (C) that can be coupled with said control ring nut (26) to control its rotation.

20. Trocar according to claim 19, **characterised in that** said key (C) has an end portion (36) engageable in the hole (26b) of said control ring nut (26), and at least one side abutment portion (37) that interacts with said control appendage (35) of said ratchet mechanism and with said at least one relief (26a) of said ring nut (26), said key (C) having means (38) for distinguishing the grip side for the opening of the trocar from the grip side for the closing.

## Patentansprüche

1. Trokar für die Thoraxchirurgie, enthaltend eine Führungsleitung (T) für chirurgische Instrumente, ausgelegt, um in eine Körperöffnung eines Zugangs zur Thoraxhöhle eingeführt zu werden, wobei die Leitung (T) durch eine Mehrzahl von Sektoren (11) gebildet ist, die um ihre Achse (12) angeordnet und an einem Halterahmen (13) angelenkt sind, wobei Sektorexpansionseinrichtungen (14) mit der Leitung (T) verbunden sind, um die Sektoren (11) wechselseitig von einer initialen Einführposition in die Körperöffnung in eine finale maximale Expansionsposition zu verstellen, in welcher sie von der Achse (12) in einem größeren Ausmaß weg beweg sind als in der Konfiguration, die in der initialen Position eingenommen wird, wobei die Expansionseinrichtungen (14) eine Steuerringbuchse (26) enthalten, die beweglich mit den Sektoren (11) und dem Halterahmen (13) verbunden ist, so dass eine Drehung der Ringbuchse (26) in einer Richtung, die die Expansionsrichtung genannt wird, zu einer Bewegung der Sektoren (11) weg von der Achse (12) führt, **dadurch gekennzeichnet, dass** Einrichtungen (29) zum Sperren der Drehung der Ringbuchse (26) in der Richtung entgegengesetzt zu jener der Expansionsrichtung mit der Ringbuchse (26) verbunden sind, wobei die Drehsperreinrichtungen (29) eine Vorrichtung (32) zum temporären Entfernen der Drehhinderung der Ringbuchse (26) in der Richtung entgegengesetzt zur Expansionsrichtung enthalten, wodurch sich die Sektoren (11) zurück zu ihrer initialen Position bewegen können.

2. Trokar nach Anspruch 1, **dadurch gekennzeichnet, dass** die Drehsperreinrichtungen (29) der Steuerringbuchse (26) in der Expansionsrichtung einen Rastmechanismus enthalten, der zwischen dem Rahmen (13) und der Steuerringbuchse (26) eingefügt ist.

3. Trokar nach Anspruch 2, **dadurch gekennzeichnet, dass** der Rastmechanismus einen gezahnten Teil (30) mit an dem Rahmen (13) ausgebildetem Sägezahnprofil, enthält, wobei eine Klinke (31) elastisch mit der Ringbuchse (26) verbunden ist, wobei ein Ende der Klinke (31) frei ist, um sich an dem gezahnten Teil (30) in der Richtung zu verschieben, die durch die Abschrägung der Zähne zugelassen ist, wobei die Vorrichtung (32) zum temporären Entfernen der Drehhinderung der Ringbuchse (26) in der Richtung entgegengesetzt zur Expansionsrichtung eine Ausrückvorrichtung der Klinke (31) aus dem gezahnten Teil (30) enthält.

4. Trokar nach Anspruch 3, **dadurch gekennzeichnet, dass** der Rahmen (13) intern einen Drehgehäuseraum für die Steuerringbuchse (26) definiert, wobei der gezahnte Teil (30) mit Sägezahnprofil an der Innenseitenoberfläche (25) des Gehäuseraums für die Steuerringbuchse (26) ausgebildet ist, wobei die Klinke (31) durch eine elastische Platte gebildet ist, mit einem Ende an der zylindrischen Flanke der Steuerringbuchse (26) befestigt und mit dem anderen Ende frei, um sich an dem gezahnten Teil (30) in der Richtung zu verschieben, die durch die Abschrägung der Zähne zugelassen ist, wobei die Vorrichtung (32) zum Ausrücken der Klinke (31) aus dem gezahnten Teil (30) eine Zunge (34) enthält, die verschiebbar zwischen der zylindrischen Flanke der Steuerringbuchse (26) und der Innenseitenoberfläche (25) des Rahmens (13) angeordnet ist, wobei die Zunge (34) ausgelegt ist, um zwischen das freie Ende der elastischen Platte, die die Klinke (31) bildet, und dem gezahnten Teil (30) gemäß der Winkelposition längs der zylindrischen Flanke der Steuerringbuchse (26) eingefügt zu werden, womit das Ausrücken der Klinke (31) zugelassen ist.

5. Trokar nach Anspruch 4, **dadurch gekennzeichnet, dass** die Steuerringbuchse (26) wenigstens ein Relief (26a) hat, das entweder ein Griffelement für die Hand des Chirurgen oder ein Anlageelement für einen Betätigungsschlüssel (C) der Steuerringbuchse (26) bildet, wobei sich das wenigstens eine Relief (26a) in der Richtung entgegengesetzt zur Ausdehnungsrichtung der Sektoren (11) erstreckt, wobei die Ausrückzunge (34) der Klinke (31) des Rastmechanismus einen Steuerfortsatz (35) hat, der an dem wenigstens einen Relief (26a) vorgesehen und dem letzteren (26a) zugewandt ist, wobei die Breite des Steuerfortsatzes (35) größer als die Breite des entsprechenden wenigstens einen Reliefs (26a) ist.

6. Trokar nach einem der vorhergehenden Ansprüche, **dadurch gekennzeichnet, dass** jeder der Sektoren (11) einen länglichen Körper (15) enthält, der in derselben Richtung der Achse (12) ausgedehnt ist, wobei sich ein Arm (16), der an einem freien Ende an dem Rahmen (13) mittels eines Scharnierstiftes (17) angelenkt ist, von einem Ende des länglichen Körpers (15) aus in einer Ebene erstreckt, die im Wesentlichen orthogonal zu der Achse (12) ist.

7. Trokar nach Anspruch 6, **dadurch gekennzeichnet, dass**, wenn die Sektoren (11) in der initialen Einführposition sind, die länglichen Körper (15) eine zylindrische Leitung bilden, die längs ihrer eigenen Seitenoberfläche im Wesentlichen geschlossen ist.

8. Trokar nach Anspruch 6 oder 7, **dadurch gekennzeichnet, dass** jeder Arm (16) mit einer zu der Achse (12) hin gewandten Konkavität gekrümmt ist.

9. Trokar nach einem der Ansprüche 6 bis 8, **dadurch gekennzeichnet, dass** der Rahmen (13) ringartig ist und die Sektoren (11) umgibt, wobei der Rahmen (13) enthält
- ein erstes ringartiges Element (18), das eine innere Basis (19), an welcher die Arme (16) der Sektoren (11) verschiebbar anliegen, und eine laterale Containmentseite (20) für die Arme hat,
- ein zweites ringartiges Element (21), das an dem oberen Rand (22) der Seite (20) des ersten ringartigen Elementes (18) befestigt ist, woran (21) Scharnierstifte (17) für die Arme (16) angebunden sind,
- ein drittes ringartiges Element (24), das an dem zweiten ringartigen Element (21) an dem Teil entgegengesetzt dem ersten ringartigen Element (18) befestigt ist, wobei das dritte ringartige Element (24) eine zylindrische Innenseitenoberfläche (25) hat, die einen Drehgehäuseraum für die Steuerringbuchse (26) definiert,
- ein viertes ringartiges Element (27), das an dem Rand des dritten ringartigen Elementes (24) an der Seite entgegengesetzt dem zweiten ringartigen Element (21) befestigt ist, wobei die Steuerringbuchse (26) in der Bewegung längs der Achse (12) zwischen dem zweiten ringartigen Element (21) und dem vierten ringartigen Element (27) gesperrt ist.

10. Trokar nach Anspruch 9, **dadurch gekennzeichnet, dass** die Verbindung des ersten, zweiten, dritten und vierten ringartigen Elementes (18, 21, 24, 27), womit der Rahmen (13) gebildet ist, durch zwei entgegengesetzte Schrauben (39) erzielt wird, die durch entsprechende Ösen (40) hindurch gehen, die peripher an denselben ringartigen Elementen (18, 21, 24, 27) ausgebildet sind.

11. Trokar nach einem der Ansprüche 4 und 9, **dadurch gekennzeichnet, dass** der gezahnte Teil (30) des Rastmechanismus der Innenseitenoberfläche (25) des dritten ringartigen Elementes (24) des Rahmens (13) ausgebildet ist.

12. Trokar nach einem der Ansprüche 6 bis 10, **dadurch gekennzeichnet, dass** die Expansionseinrichtungen (14) Stifte (28) enthalten, die von der Oberfläche der Steuerringbuchse (26) bis zum Kontakt mit den inneren Flanken (16a) der Arme (16) ausgedehnt sind, wobei die Stifte (28) an den Flanken (16a) verschiebbar sind, um die Öffnungsbewegung der Sektoren von der initialen Einführposition in die Körperöffnung zum Zugang zu der Thoraxhöhle zu der finalen maximalen Expansionsposition zu gestatten.

13. Trokar nach Anspruch 12, **dadurch gekennzeichnet, dass** an den inneren Flanken (16a) der Arme (16), an den Anbringendzonen der Arme (16) an den länglichen Körpern (15), die die Sektoren (11) bilden, Endstoppanlagen (16b) für die Stifte (28) gebildet sind, wenn die Sektoren (11) in der maximalen Expansionsposition sind.

14. Trokar nach einem der vorhergehenden Ansprüche, **dadurch gekennzeichnet, dass** er Befestigungseinrichtungen an dem Patiententhorax enthält.

15. Trokar nach Anspruch 14, **dadurch gekennzeichnet, dass** die Befestigungseinrichtungen längs der Außenseite des Rahmens (13) ausgebildete kleine Ringe (46) enthalten, die ausgelegt sind, um den Durchgang von Nahtpunkten zum Verankern an der Patientenhaut zuzulassen.

16. Trokar nach einem oder mehreren der vorhergehenden Ansprüche, **dadurch gekennzeichnet, dass** er ein Zugangselement enthält, das durch ein Einführwerkzeug (U) gebildet ist, das ausgelegt ist, um die Expansion der Sektoren (11) in der initialen Einführposition zu sperren, wobei das Einführwerkzeug (U) mit dem Trokarkörper (10) nur in dem Einführschritt in die Körperöffnung verbindbar ist und nachfolgend entfernt wird, bevor der Expansionsschritt begonnen wird.

17. Trokar nach Anspruch 16, **dadurch gekennzeichnet, dass** das Einführwerkzeug (U) eine Platte (41) hat, die zum Loch (26b) der Steuerringbuchse (26) einen Gegenform hat und passt, um darin (26b) eingesetzt zu werden, wobei die Platte (41) auf der Seite, die den Sektoren (11) zugewandt ist, Stifte (47) hat, die ausgelegt sind, um gegen die externen Flanken (16c) anzuliegen, die entgegengesetzt zu den internen Flanken (16a), an welchen sich die Stifte (28) der Steuerringbuchse (26) verschieben, von entsprechenden Armen (16) sind, wobei das Einführwerkzeug (U) an der Steuerringbuchse (26) mittels eines Verriegelungs- und Positionierelementes verriegelt ist, das von der Seite der Platte (41) vorsteht und ausgelegt ist, um mit einer entsprechenden Nut (42) gekoppelt zu werden, die an der Innenseitenoberfläche der Steuerringbuchse (26) ausgebildet ist.

18. Trokar nach Anspruch 17, **dadurch gekennzeichnet, dass** sich ein stabähnlicher Teil (43) von der Platte (41) in den Raum erstreckt, der zwischen den länglichen Körpern (15) definiert ist, die die Sektoren (11) bilden, wobei der stabähnliche Teil von der Leitung, die von diesen (11) gebildet wird, mit einem geformten Ende (44) vorsteht, um die Einführung des Trokars in die Körperöffnung zu erleichtern.

19. Trokar nach einem der vorhergehenden Ansprüche, **dadurch gekennzeichnet, dass** er ein Zugangselement enthält, das durch einen Schlüssel (C) gebildet ist, der mit der Steuerringbuchse (26) gekoppelt werden kann, um ihre Drehung zu steuern.

20. Trokar nach Anspruch 19, **dadurch gekennzeichnet, dass** der Schlüssel (C) einen Endteil (36), der in dem Loch (26b) der Steuerringbuchse (26) in Eingriff bringbar ist, und wenigstens einen Seitenanlagenteil (37) hat, der mit dem Steuerfortsatz (35) des Rastmechanismus und mit dem wenigstens einen Relief (26a) der Steuerringbuchse (26) interagiert, wobei der Schlüssel (C) Einrichtungen (38) hat, um die Griffseite für das Öffnen des Trokars von der Griffseite für das Schließen zu differenzieren.

## Revendications

1. Trocart pour chirurgie thoracique, comprenant un conduit guide (T) pour instruments chirurgicaux apte à être inséré dans une incision réalisée dans un corps pour accéder à la cavité thoracique, ledit conduit (T) étant constitué d'une pluralité de secteurs (11) disposés autour de son axe (12) et articulés sur un cadre support (13), des moyens d'expansion (14) des secteurs étant associés au dit conduit (T) pour déplacer mutuellement lesdits secteurs (11) depuis une position d'insertion initiale dans l'incision réalisée dans le corps jusqu'à une position d'expansion finale maximum dans laquelle ils sont éloignés du dit axe (12) en une extension plus grande que dans la configuration prise dans ladite position initiale, lesdits moyens d'expansion (14) comprenant un embout de commande en anneau (26) relié de façon mobile aux dits secteurs (11) et au dit cadre support (13), de façon qu'une rotation du dit embout en anneau (26) dans un sens, appelé sens d'expansion, a comme conséquence un mouvement des secteurs (11) les éloignant du dit axe (12), ***caractérisé en ce que*** des moyens de blocage de la rotation du dit embout en anneau (26) dans le sens opposé au sens d'expansion sont associés au dit embout en anneau (26), lesdits moyens de blocage en rotation (29) comprenant un dispositif (32) pour supprimer temporairement l'obstacle à la rotation de l'embout en anneau (26) dans le sens opposé au sens d'expansion, de façon que les secteurs (11) puissent se déplacer en arrière vers ladite position initiale.

2. Trocart selon la revendication 1, **caractérisé en ce que** les moyens de blocage en rotation (29) du dit embout de commande en anneau (26) dans le sens d'expansion comportent un mécanisme à rochet interposé entre ledit cadre (13) et ledit embout de commande en anneau (26).

3. Trocart selon la revendication 2, **caractérisé en ce que** ledit mécanisme à rochet comporte une portion dentée (30) avec un profil en dents de scie formée sur ledit cadre, un cliquet (31) élastiquement lié au dit embout de commande en anneau (26), une extrémité du cliquet (31) étant libre de glisser sur ladite portion dentée (30) dans le sens autorisé par la pente des dents, ledit dispositif (32) pour supprimer temporairement l'obstacle à la rotation de l'embout en anneau (26) dans le sens opposé au sens d'expansion comprenant un dispositif de dégagement du dit cliquet (31) pour le dégager de ladite portion dentée (30).

4. Trocart selon la revendication 3, **caractérisé en ce que** ledit cadre (13) définit intérieurement un espace de logement rotationnel pour ledit embout de commande en anneau (26), ladite portion dentée (30) avec un profil en dents de scie étant formé sur la surface latérale intérieure (25) du dit espace de logement pour ledit embout de commande en anneau (26), ledit cliquet (31) étant constitué d'une lame élastique ayant une extrémité fixée sur le flanc cylindrique du dit embout de commande en anneau (26) et l'autre extrémité libre de glisser sur ladite portion dentée (30) dans le sens autorisé par la pente des dents, ledit dispositif (32) pour dégager le cliquet (31) de la portion dentée (30) comprenant une languette (34) montée de façon coulissante entre le flanc cylindrique de l'embout de commande en anneau (26) et ladite surface latérale intérieure (25) du cadre (13), ladite languette (34) étant adaptée pour être interposée entre l'extrémité libre de la lame élastique formant le cliquet (31) et ladite portion dentée (30) selon la position angulaire le long du flanc cylindrique de l'embout de commande en anneau (26), permettant de ce fait le dégagement du cliquet (31).

5. Trocart selon la revendication 4, **caractérisé en ce que** ledit embout de commande en anneau (26) comporte au moins un ergot en saillie (26a) constituant soit un élément de préhension pour la main du chirurgien soit un élément de butée pour une clé d'entraînement (C) de l'embout en anneau (26), ledit au moins un ergot en saillie (26a) s'étendant dans le sens opposé au sens d'extension des dits secteurs (11), ladite languette de dégagement (34) du cliquet (31) du mécanisme à rochet ayant un appendice de commande (35) prévu sur ledit au moins un ergot en saillie (26a) et faisant face à ce dernier (26a), la largeur du dit appendice de commande (35) étant plus grande que la largeur du dit au moins un ergot en saillie correspondant (26a).

6. Trocart selon l'une quelconque des revendications précédentes, **caractérisé en ce que** chacun des dits secteurs (11) comporte un corps longitudinal (15) qui s'étend dans la même direction que ledit axe (12), un bras (16) articulé à une extrémité libre du dit cadre (13) au moyen d'une goupille charnière (17) s'étendant à partir d'une extrémité du dit corps longitudinal (15), dans un plan sensiblement orthogonal au dit axe (12).

7. Trocart selon la revendication 6, **caractérisé en ce que**, quand lesdits secteurs (11) sont dans la position d'insertion initiale, lesdits corps longitudinaux (15) forment un conduit cylindrique qui est sensiblement fermé le long de sa propre surface latérale.

8. Trocart selon l'une des revendications 6 et 7, **caractérisé en ce que** chaque bras (16) est courbé avec une concavité tournée vers ledit axe (12).

9. Trocart selon l'une quelconque des revendications 6 à 8, **caractérisé en ce que** ledit cadre (13) est annulaire et entoure lesdits secteurs (11), ledit cadre (13) comprenant :
- un premier élément annulaire (18) ayant une embase intérieure (19) sur laquelle viennent buter de façon coulissante les bras (16) des dits secteurs (11), et une paroi latérale de retenue (20) pour lesdits bras (16),
- un deuxième élément annulaire (21), fixé sur le bord supérieur (22) de ladite paroi (20) du dit premier élément annulaire (18), auquel (21) sont liées des goupilles charnières (17) pour les bras (16),
- un troisième élément annulaire (24) fixé sur ledit deuxième élément annulaire (21) sur la partie opposée au premier élément annulaire (18), ledit troisième élément annulaire (24) ayant une surface latérale intérieure cylindrique (25) qui définit un espace de logement rotationnel pour ledit embout de commande en anneau (26),
- un quatrième élément annulaire (27) fixé sur le bord du troisième élément annulaire (24) sur le côté opposé au deuxième élément annulaire (21), ledit embout de commande en anneau (16) étant bloqué en mouvement le long de l'axe (12) entre le deuxième élément annulaire (21) et le quatrième élément annulaire (27).

10. Trocart selon la revendication 9, **caractérisé en ce que** l'assemblage des dits premier, deuxième, troisième et quatrième éléments annulaires (18, 21, 24, 27) formant le cadre (13) est obtenu par deux vis en opposition (39) passant à travers des oeillets correspondants (40) réalisés à la périphérie sur ces mêmes éléments annulaires (18, 21, 24, 27).

11. Trocart selon l'une des revendications 4 et 9, **caractérisé en ce que** ladite portion dentée (30) du mécanisme à rochet est formée sur la surface latérale intérieure (25) du dit troisième élément annulaire (24) du cadre (13).

12. Trocart selon l'une quelconque des revendications 6 à 10, **caractérisé en ce que** lesdits moyens d'expansion (14) comprennent des doigts (28) qui s'étendent à partir de la surface du dit embout de commande en anneau (26) jusqu'au contact avec les flancs intérieurs (16a) des dits bras (16), lesdits doigts (28) étant montés coulissants sur lesdits flancs (16a) pour permettre le mouvement d'ouverture des dits secteurs (11) à partir de la position d'insertion initiale dans l'incision dans le corps pour accéder à la cavité thoracique jusqu'à leur position d'expansion maximum finale.

13. Trocart selon revendication 12, **caractérisé en ce que** sont réalisées, sur lesdits flancs intérieurs (16a) des dits bras (16), dans la zone d'extrémité d'attache des bras (16) sur les corps longitudinaux (15) formant lesdits secteurs (11), des butées d'arrêt d'extrémité (16b) pour lesdits doigts (28) quand les secteurs (11) sont en position d'expansion maximum.

14. Trocart selon l'une quelconque des revendications précédentes, **caractérisé en ce qu'**il comporte des moyens de fixation sur le thorax du patient.

15. Trocart selon la revendication 14, **caractérisé en ce que** lesdits moyens de fixation comportent des petits anneaux (46) réalisés à l'extérieur et le long du dit cadre (13) adaptés pour permettre le passage de points de suture pour l'ancrage sur la peau du patient.

16. Trocart selon l'une ou plusieurs des revendications précédentes, **caractérisé en ce qu'**il comporte un élément accessoire constitué par un outil d'insertion (U) adapté pour bloquer l'expansion des dits secteurs (11) dans leur position d'insertion initiale, ledit outil d'insertion (U) pouvant être associé à l'ensemble du trocart (10) seulement lors de l'étape d'insertion dans l'incision dans le corps et étant ensuite enlevé avant de commencer l'étape d'expansion.

17. Trocart selon la revendication 16, **caractérisé en ce que** ledit outil d'insertion (U) comporte une plaque (41) de forme complémentaire à celle de l'orifice (26b) du dit embout de commande en anneau (26) et agencée pour y être insérée (26b), ladite plaque (41) ayant, sur la face tournée vers lesdits secteurs (11), des ergots (47) adaptés pour buter contre les flancs extérieurs (16c) des bras respectifs (16), opposés aux flancs intérieurs (16a) sur lesquels coulissent les doigts (28) du dit embout de commande en anneau (26), ledit outil d'insertion (U) étant bloqué sur ledit embout de commande en anneau (26) au moyen d'un élément de blocage et de positionnement s'étendant à partir du côté de ladite plaque (41) et adapté pour être monté en coopération avec une gorge correspondante (42) réalisée sur la surface latérale intérieure du dit embout de commande en anneau (26).

18. Trocart selon revendication 17, **caractérisé en ce que**, une partie en forme de tige (43) s'étend à partir de ladite plaque (41) dans l'espace défini entre les corps longitudinaux (15) formant lesdits secteurs (11), ladite partie en forme de tige s'étendant à partir du conduit formé par ces derniers (11) avec une extrémité conformée (44) pour rendre plus facile l'insertion du trocart dans l'incision dans le corps.

19. Trocart selon l'une quelconque des revendications précédentes, **caractérisé en ce qu'**il comprend un élément accessoire formé d'une clé (C) qui peut être couplée au dit embout de commande en anneau (26) pour commander sa rotation.

20. Trocart selon la revendication 19, **caractérisé en ce que** ladite clé (C) présente une partie d'extrémité (36) pouvant être engagée dans l'orifice (26b) du dit embout de commande en anneau (26), et au moins une partie de butée latérale (37) qui coopère avec ledit appendice de commande (35) du dit mécanisme à rochet et avec le au moins un ergot en saillie (26a) du dit embout en anneau (26), ladite clé (C) ayant des moyens (38) pour distinguer le côté de préhension pour l'ouverture du trocart, du côté de préhension pour sa fermeture.
